# FASCICULE DE BREVET EUROPEEN

(11) **EP 3 419 530 B1**
(45) Date de publication et mention de la délivrance du brevet: **07.10.2020**
(21) Numéro de dépôt: 17710343.9
(22) Date de dépôt: 22.02.2017
(51) Int. Cl.: A61B 17/16, A61B 46/10, A61B 10/02

(54) **DISPOSITIF POUR LE COUPLAGE STÉRILE D'UN INSTRUMENT CHIRURGICAL PERCUTANÉ ET D'UN OUTIL D'ENTRAINEMENT ET MÉTHODE POUR RÉALISER UN TEL COUPLAGE**
VORRICHTUNG ZUR STERILEN KOPPLUNG EINES PERKUTANEN CHIRURGISCHEN INSTRUMENTS UND ANTRIEBSWERKZEUG UND VERFAHREN ZUR DURCHFÜHRUNG SOLCH EINER KOPPLUNG
DEVICE FOR THE STERILE COUPLING OF A PERCUTANEOUS SURGICAL INSTRUMENT AND A DRIVING TOOL AND METHOD FOR CARRYING OUT SUCH A COUPLING

(30) Priorité: 22.02.2016 FR 1651435
(43) Date de publication de la demande: 02.01.2019
(73) Titulaire: Fumex, Laurent, Madison, CT 06443 (US); Masseglia, Thierry, 83130 La Garde (FR)
(72) Inventeur: Fumex, Laurent, Madison, CT 06443 (US); Masseglia, Thierry, 83130 La Garde (FR)
(74) Mandataire: Ipsilon
(86) Numéro de dépôt international: PCT/FR2017/050391
(87) Numéro de publication internationale: WO 2017/144816

(56) Documents cités:
- WO-A1-2015/154188
- WO-A1-2017/046531
- US-A1- 2006 142 881
- US-A1- 2008 045 860
- US-A1- 2009 194 446

## Description

### DOMAINE DE L'INVENTION

L'invention se rapporte au domaine général de l'instrumentation chirurgicale percutanée. Elle se rapporte plus particulièrement aux instruments chirurgicaux percutanés destinés à traverser l'épiderme et les parties molles du corps afin d'effectuer, par exemple, des prélèvements de moelle et de tissus osseux à des fins de biopsie.

### CONTEXTE DE L'INVENTION - ART ANTERIEUR

Dans le domaine de la chirurgie percutanée et plus particulièrement celui de la biopsie osseuse percutanée, il est connu d'utiliser différents instruments permettant à la fois de pénétrer les tissus mous puis la corticale de l'os concerné et de réaliser le prélèvement de substances organiques (moelle osseuse, échantillons osseux) contenues dans l'os ou dans sa cavité.

Ces instruments sont généralement connus sous l'appellation d'aiguilles à biopsie osseuse ou de moelle et sont généralement composés d'un trocart ou aiguille d'accès, combiné à une canule biopsique. De nature plus ou moins perforante, les trocarts ou aiguilles d'accès permettent au praticien de traverser les tissus mous puis de forer l'os considéré de façon à atteindre une zone où une biopsie osseuse doit être effectuée.

Ces trocarts comportent généralement un tube externe creux, dont l'extrémité est plus ou moins coupante, et à l'intérieur duquel coulisse une tige dont l'extrémité est affûtée de façon à perforer l'os, cette tige étant éventuellement configurée pour prélever un échantillon du tissu qui se trouve dans la cavité.

De tels instruments sont généralement mis en oeuvre de manière manuelle au moyen d'une poignée solidarisée, de manière permanente ou non, à l'extrémité proximale (extrémité n'entrant pas en contact avec l'os) de la tige perforante, que le praticien actionne en rotation de façon à forer l'os. En pratique, le praticien amène l'extrémité du tube externe du trocart au contact de l'os puis actionne la poignée en rotation pour faire pénétrer l'extrémité de la tige dans l'os.

Ainsi, la demande Internationale publiée sous la référence WO2006/061514 décrit un trocart destiné à la biopsie osseuse comprenant un tube externe, dont l'extrémité distale est divisée en deux segments à bord coupant hélicoïdal, dans lequel coulisse une tige affûtée. Ce type d'instrument est destiné à être mis en œuvre manuellement au moyen d'une poignée.

La mise en œuvre manuelle d'un tel trocart, au moyen d'une poignée directement actionnée par le praticien, correspond à un mode opératoire classique qui permet à ce dernier de maitriser de manière fine le geste de perforation de l'os. Cependant, dans certaines circonstances, en particulier lorsque la dureté de l'os requiert une plus grande vigueur pour faire pénétrer l'aiguille à travers la paroi de l'os, il est alors parfois plus confortable, tant pour le patient que pour le praticien, de disposer d'un outil d'entrainement, de type perceuse, pour faire pénétrer la tige à l'intérieur de l'os sans appliquer une force excessive.

Par suite il y a un grand avantage pour le praticien à pouvoir disposer d'un trocart dont la tige perforante peut être actionnée indifféremment au moyen d'une simple poignée ou par l'intermédiaire d'un outil d'entrainement.

Une telle possibilité permet avantageusement au praticien, de débuter une procédure opératoire manuellement puis, si la dureté de l'os le nécessite, de terminer la perforation au moyen d'un entrainement automatique en rotation et ce, sans qu'il soit nécessaire de changer la tige perforante et donc sans risque de perte du point d'entrée dans l'os.

Elle nécessite cependant de disposer d'un dispositif d'interface capable par une de ses extrémités de venir se substituer à la poignée utilisée par le praticien pour une mise en œuvre manuelle du trocart ; en se montant à l'extrémité de ce dernier de manière identique à la poignée, et portant à son autre extrémité un élément d'interface sur lequel l'embout d'un outil d'entrainement puisse venir se fixer de manière amovible.

Le dispositif envisagé ici joue ainsi le rôle d'un élément de couplage qui permet d'entrainer le trocart en rotation au moyen d'un outil.

Un tel dispositif doit, entre autres fonctionnalités, pouvoir assurer un positionnement axial relativement précis de l'outil d'entrainement vis-à-vis du trocart, faute de quoi la précision de pénétration du trocart pourrait se trouver affectée.

Une telle possibilité nécessite également, dans la mesure où le dispositif d'interface est destiné à assurer le raccordement de l'extrémité d'un trocart, objet stérile et généralement à usage unique, à un outillage de type perceuse, objet naturellement réutilisable et généralement non stérile, que le dispositif d'interface puisse être associé de manière simple à des moyens permettant le montage de l'outil d'entrainement à l'extrémité du trocart et l'entrainement en rotation dudit trocart en évitant tout contact entre le trocart et l'outil d'entrainement, de nature à provoquer la contamination de l'un par l'autre.

La demande de brevet français publiée sous la référence FR 3007636 décrit ainsi un système d'interface de structure simple constitué d'un élément mâle hexagonal monté à l'extrémité proximale du trocart et d'un élément hexagonal femelle dans lequel l'élément mâle vient se loger, l'ensemble étant solidarisé de manière amovible par encliquetage.

Ce document décrit également que, selon le moyen d'entrainement en rotation considéré, l'élément hexagonal femelle est soit directement intégré à la poignée (cas d'une mise en rotation manuelle du trocart), soit intégré à l'extrémité d'un dispositif d'interface directement monté sur l'arbre de sortie de l'outil d'entrainement en rotation (perceuse).

Dans les deux cas l'élément hexagonal femelle conserve avantageusement des caractéristiques morphologiques semblables (tronçon polygonale des éléments mâle et femelle) de sorte que l'extrémité du trocart peut s'emboiter indifféremment dans la poignée ou dans l'extrémité de l'outil.

Cette demande de brevet décrit ainsi un système d'interface simple et peu onéreux, permettant de relier un trocart à un moyen permettant d'actionner ce dernier, poignée ou outil d'entrainement en rotation indifféremment. La liaison réalisée est une liaison amovible permettant un alignement axial précis de l'axe de rotation imposé par l'actionneur et de l'axe du trocart. Cependant, le dispositif de couplage décrit dans cette demande ne permet pas, en l'état, de réaliser un couplage stérile entre l'actionneur et le trocart.

Le brevet US6716215 décrit un moyen offrant une solution pour raccorder une mèche stérile à une perceuse non stérile au travers d'un sachet stérile. Le sachet stérile, sur lequel est fixée une bague, est destiné à recevoir une perceuse qu'il permet d'emballer pour pouvoir l'utiliser dans un milieu stérile.

Dans la zone de raccordement de la perceuse à la mèche, l'étanchéité est réalisée au moyen d'un joint torique, logé dans la bague, qui fait étanchéité sur le corps de la mèche. L'étanchéité est réalisée par compression du joint.

Cette solution présente cependant l'inconvénient que le sachet peut se trouver entrainé en rotation du fait des frottements du joint d'étanchéité sur la mèche et qu'il peut donc, en tournant, bloquer la perceuse en rotation.

Le brevet US7850620 décrit un système permettant de raccorder une perceuse, non stérile logée dans un sachet stérile, à un instrument de biopsie stérile au moyen d'un coupleur solidaire du sachet. Le coupleur comporte une bague bloquée en translation mais libre en rotation sur laquelle est soudé le sachet. Cette solution ne permet pas de réaliser une étanchéité complète car la bague peut laisser passer des impuretés de par son jeu de fonctionnement. Par ailleurs un tel dispositif n'exclut pas le risque que compte-tenu de la structure du coupleur, le sachet ne soit entrainé en rotation sous l'effet de frottements et ne bloque ainsi la perceuse en rotation.

La demande de brevet publiée sous la référence WO 2015/154188 décrit également un système permettant de raccorder une perceuse, non stérile logée dans un sachet stérile, à un instrument stérile au moyen d'une bague solidaire du sachet et d'un axe d'accouplement monté dans la bague. La bague est formée par deux anneaux concentriques vissés l'un à l'autre de manière à enserrer le sachet au niveau d'une ouverture de ce dernier permettant le passage de l'axe d'accouplement au travers du sachet. L'étanchéité entre l'extérieur et l'intérieur du sachet est réalisée par un joint d'étanchéité rapporté et logé au niveau de l'axe d'accouplement de manière à venir au contact de la bague lorsque l'axe d'accouplement coopère avec la bague.

Un inconvénient majeur de ce système réside dans le fait que la bague n'est pas prévue pour être solidarisée à la perceuse de sorte que le sachet peut être entrainé en rotation sous l'effet de frottements et bloquer ainsi la perceuse en rotation.

De plus, la conception de la bague et de l'axe d'accouplement est complexe de sorte qu'elle induit une fabrication et un assemblage couteux des pièces du système. En effet, la bague et l'axe d'accouplement comprennent de multiples pièces nécessitant un assemblage préalable au montage de l'instrument sur la perceuse, notamment par le vissage des anneaux entre eux et par le montage du roulement à bille sur l'axe d'accouplement. De plus, la mise en place du joint d'étanchéité nécessite la réalisation d'usinages sur l'axe d'accouplement et la bague ce qui entraîne un coût supplémentaire de fabrication.

Par ailleurs le document WO 2015/154188 A1 décrit un dispositif comprenant une perceuse à connecter avec un embout pour l'entraîner en rotation. Le dispositif comprend également un sac solidaire d'une bague apte à coopérer avec l'embout.

### PRESENTATION DE L'INVENTION

Un but de l'invention est de proposer une solution pour raccorder une mèche stérile à une perceuse non stérile au travers d'un sachet stérile, remédiant au moins en partie aux inconvénients précités.

A cet effet l'invention a pour objet un dispositif de couplage stérile d'un instrument chirurgical percutané à un outil d'entrainement en rotation dudit instrument suivant l'objet de la revendication 1 comportant une extrémité munie d'un embout de couplage. Ce dispositif comporte :
- une enveloppe souple présentant une première ouverture de forme circulaire et une seconde ouverture permettant d'introduire l'outil d'entrainement dans ladite enveloppe. La première ouverture est positionnée sur la paroi de l'enveloppe de telle façon que, lorsque l'outil d'entrainement se trouve dans la poche, l'embout de couplage se trouve placé en regard de ladite première ouverture;
- un embout de liaison, comportant un tronçon distal cylindrique assurant le couplage avec l'instrument chirurgical percutané et un tronçon proximal assurant le couplage avec l'outil d'entrainement. L'embout de

liaison est configuré pour traverser l'enveloppe par la première ouverture circulaire de telle sorte que l'extrémité de son tronçon distal soit placée à l'extérieur de l'enveloppe, son tronçon proximal étant placé à l'intérieur de l'enveloppe;
- une bague tubulaire formant un conduit dans laquelle l'embout de liaison est destiné à être inséré. La bague est positionnée sur la surface interne de l'enveloppe en regard de la première ouverture circulaire. Elle est solidaire de l'enveloppe par une de ses extrémités, son autre extrémité étant configurée pour solidariser l'enveloppe à la paroi de l'outil d'entrainement par insertion de l'extrémité du corps de l'outil d'entrainement dans le conduit.

Le conduit est configuré de telle façon que l'embout de liaison puisse tourner librement autour de son axe à l'intérieur de la bague tout en étant maintenu axialement;

Selon l'invention, l'enveloppe, la bague et l'embout de liaison comportant des moyens qui coopèrent pour former une barrière étanche au niveau de la première ouverture circulaire, ladite barrière étant configurée de façon à ne pas entraver la rotation de l'embout de liaison.

Selon diverses dispositions pouvant être considérées séparément ou en groupe le dispositif selon l'invention peut présenter les caractéristiques qui suivent.

Selon une caractéristique particulière, le tronçon distal de l'embout de liaison présente une forme tubulaire et comporte une empreinte configurée pour accueillir l'extrémité proximale d'un instrument chirurgical percutané. L'extrémité du tronçon proximal de l'embout de liaison est configurée pour pouvoir se loger dans l'embout de couplage de l'outil d'entrainement.

Selon une autre caractéristique, la bague comporte un flasque en forme de disque situé à l'extrémité de la partie tubulaire par laquelle ladite bague est fixée sur la paroi interne de l'enveloppe, le flasque présentant une ouverture centrale dont le centre est situé sur l'axe de symétrie de la partie tubulaire. La bague et le flasque sont fixés sur la paroi interne de l'enveloppe de telle façon que le centre de la première ouverture soit également aligné sur l'axe de symétrie de la partie tubulaire.

Selon une autre caractéristique, la bague présentant les caractéristiques précédentes, l'embout de liaison comporte en outre une protubérance annulaire positionnée sur sa surface externe et délimitant le tronçon distal et le tronçon proximal; le diamètre extérieur de la protubérance annulaire étant sensiblement égale, à un jeu fonctionnel près, au diamètre intérieur du corps tubulaire de la bague et supérieur au diamètre de l'ouverture du flasque, de telle sorte que l'embout de liaison puisse coulisser dans la bague suivant un axe sensiblement confondu avec l'axe de symétrie du corps tubulaire et traverser le flasque jusqu'à ce que la protubérance annulaire vienne en butée sur la paroi interne du flasque. L'axe de symétrie de l'embout de liaison est alors maintenu sensiblement confondu avec l'axe de symétrie du corps tubulaire par la protubérance annulaire. Le flasque, la paroi intérieure du corps tubulaire de la bague et la protubérance annulaire de l'embout de liaison, forment une chicane limitant la possibilité de passage d'éléments fluides ou solides entre l'intérieur et l'extérieur de l'enveloppe par la première ouverture.

Selon une autre caractéristique, la première ouverture de l'enveloppe souple présente un diamètre légèrement inférieur au diamètre externe du tronçon distal cylindrique de l'embout de liaison ledit diamètre étant défini de telle sorte que lorsque l'embout de liaison est inséré dans cette ouverture depuis l'intérieur de l'enveloppe, le bord de ladite première ouverture se déforme et constitue un repli annulaire dirigé vers l'extérieur de l'enveloppe qui vient entourer la surface externe du corps cylindrique du tronçon distal de l'embout. Ce repli constitue un joint qui coopère avec la chicane formée par le flasque de la bague, la paroi intérieure du corps tubulaire de la bague et la protubérance annulaire de l'embout de liaison pour constituer une barrière étanche au niveau de la première ouverture de l'enveloppe.

Selon une autre caractéristique, le corps de l'outil d'entrainement présentant une forme conique au niveau de l'extrémité munie de l'embout de couplage, l'extrémité de la bague tubulaire présente une ouverture de forme conique dimensionnée de telle façon que lorsque l'extrémité de l'outil d'entrainement est inséré dans la bague, cette insertion provoque un emmanchement serré de la bague sur la l'extrémité de l'outil d'entrainement qui bloque tout mouvement relatif en translation et/ou en rotation de l'enveloppe par rapport à l'outil d'entrainement.

Selon une autre caractéristique, la paroi de l'extrémité du corps de l'outil comportant une protubérance longitudinale formant une clavette, la paroi interne de la bague tubulaire comporte une échancrure longitudinale dimensionnée pour recevoir ladite clavette. L'ensemble clavette-échancrure forme un moyen complémentaire de blocage du mouvement relatif en en rotation de l'enveloppe par rapport à l'outil d'entrainement.

Selon une autre caractéristique, l'embout de couplage de l'outil d'entrainement formant une cavité comportant une zone arrière de forme hexagonale et une extrémité avant de forme tronconique, le tronçon proximal de l'embout de liaison forme un élément mâle de section hexagonale, dimensionné de façon à pouvoir s'insérer, avec un minimum de jeu, dans la cavité de section hexagonale de l'embout de couplage. La paroi externe du tronçon proximal de l'embout de liaison comporte une zone tronconique agencée sur ladite paroi externe et conformée pour coopérer avec l'extrémité avant de forme tronconique de l'embout de couplage, de façon à assurer le maintien de la coaxialité des deux éléments lors de l'utilisation.

Selon une autre caractéristique, la paroi interne de la cavité de l'embout de couplage de l'outil d'entrainement comportant une gorge chanfreinée au niveau de la zone arrière hexagonale, le corps du tronçon proximal de l'embout de liaison comporte des bossages chanfreinés conformés pour pouvoir se loger dans ladite gorge chanfreinée et assurer un blocage par encliquetage du corps du tronçon proximal de l'embout de liaison dans l'embout de couplage.

Selon une autre caractéristique, la cavité formée par l'embout de couplage de l'outil d'entrainement comportant un perçage cylindrique borgne formant le fond de la cavité et dont l'axe de symétrie est confondu avec l'axe de symétrie général de l'embout de couplage, le corps du tronçon proximal de l'embout de liaison comporte à son extrémité un élément d'extrémité cylindrique fendu sur sa longueur, et dont le diamètre est défini de façon à ce que son insertion dans le perçage cylindrique borgne se fasse avec un minimum de jeu.

Selon une autre caractéristique, la paroi externe de l'élément d'extrémité du corps du tronçon proximal de l'embout de liaison comporte des bossages formant des surépaisseurs telles que le diamètre de la section de l'élément d'extrémité est localement supérieur au diamètre du perçage de l'embout de couplage de l'outil d'entrainement et que l'emboitement de l'élément d'extrémité du corps du tronçon proximal de l'embout de liaison s'effectue sans jeu.

Selon une autre caractéristique, l'extrémité distale l'embout de liaison comporte une empreinte hexagonale permettant de recevoir l'extrémité proximale d'un instrument percutané chirurgical dont l'extrémité proximale comporte un embout de section hexagonale.

Selon une autre caractéristique, le corps cylindrique du tronçon distal de l'embout de liaison comporte une gorge annulaire apte à recevoir une bague repère. Ladite bague repère est agencée sur le corps cylindrique de telle façon que lorsque le couplage est effectué, si la barrière étanche au niveau de la première ouverture de l'enveloppe est effective, la bague repère est visible par le praticien.

Selon une autre caractéristique, la bague tubulaire est formée de manière monobloc.

Selon une autre caractéristique, la bague tubulaire est positionnée sur la surface interne de l'enveloppe et solidaire de l'enveloppe par une de ses extrémités de sorte que l'ensemble de la bague tubulaire est disposée à l'intérieur de l'enveloppe lorsque l'outil d'entrainement se trouve dans l'enveloppe.

L'invention a également pour objet un procédé pour réaliser le couplage stérile d'un instrument de biopsie et d'un outil d'entrainement dudit instrument au moyen d'un dispositif de couplage selon l'invention, le procédé tel que défini par l'objet de la revendication 16 comportant les étapes suivantes:
- une étape préliminaire de retournement de l'enveloppe sur elle-même, cette étape étant réalisée en repoussant la bague à l'intérieur de l'enveloppe jusqu'à ce que son extrémité libre émerge de l'enveloppe au travers de la seconde ouverture de l'enveloppe;
- une première étape durant laquelle l'assistant assemble l'embout de couplage de l'outil d'entrainement sur l'extrémité proximale de l'embout de liaison du dispositif jusqu'à réaliser l'emboitement des deux éléments l'un dans l'autre;
- une deuxième étape durant laquelle le praticien saisit et présente l'enveloppe retournée et durant laquelle l'assistant, après avoir introduit l'extrémité distale de l'outil d'entrainement dans la bague de l'enveloppe, lâche l'outil d'entrainement, celui-ci étant alors maintenu par le praticien au moyen de l'enveloppe retournée sur elle-même de telle sorte que, du fait du poids de l'outil d'entrainement, l'enveloppe reprend partiellement sa forme dépliée;
- une troisième étape durant laquelle le praticien termine de remettre en forme l'enveloppe sans toucher à l'outil d'entrainement, de façon à ce qu'elle enferme l'outil d'entrainement puis referme hermétiquement la seconde ouverture de l'enveloppe;
- une quatrième étape durant laquelle le praticien met en place l'instrument chirurgical percutané en insérant l'embout de liaison dudit instrument dans l'extrémité de l'embout de liaison.

Selon une disposition particulière, le dispositif de couplage étant livré au praticien sous emballage stérile avec son enveloppe pré-retournée, l'étape préliminaire est implémentée, de manière indépendante, au moment du conditionnement du dispositif de couplage.

### DESCRIPTION DES FIGURES

Les caractéristiques et avantages de l'invention seront mieux appréciés grâce à la description qui suit, description qui s'appuie sur les figures annexées qui présentent:
- la figure 1, une vue partielle en coupe, du dispositif de couplage selon l'invention, prise au niveau de la zone d'insertion de l'extrémité d'un instrument chirurgical dans l'embout de liaison du dispositif;
- les figures 2 et 3, des vues schématiques en perspective illustrant un exemple d'outil d'entrainement équipé d'un embout permettant son couplage avec un instrument de biopsie;
- la figure 4, une vue schématique en perspective de l'enveloppe du dispositif de couplage selon l'invention dans une forme de réalisation particulière, adaptée notamment à l'outil d'entrainement des figures 2 et 3;
- les figures 5 et 6, des vues schématiques en perspective illustrant la bague de l'enveloppe du dispositif de couplage selon l'invention dans la forme de réalisation correspondant à la figure 4;
- les figures 7 à 9, des vues schématiques en perspective présentant l'embout de liaison du dispositif de couplage selon l'invention, dans la forme de réalisation considérée;
- la figure 10, une vue schématique en perspective d'une bague repère destinée à équiper le dispositif selon l'invention, dans la forme de réalisation considérée;

Il est à noter que, sur les différentes figures, un même élément fonctionnel ou structurel est toujours identifié par le même renvoi au texte.

### DESCRIPTION DETAILLEE

Pour des raisons de clarté de l'exposé des différents aspects de l'invention, la description qui suit présente les différentes caractéristiques du dispositif de couplage selon l'invention au travers d'une forme de réalisation particulière prise comme exemple et de son utilisation pour réaliser le couplage stérile d'un instrument de biopsie à un outil d'entrainement.

Il est cependant entendu que la portée ou l'étendue de l'invention est seulement limitée par les combinaisons de caractéristiques objets des revendications et n'est nullement limitée à ces seuls exemples de réalisation et d'utilisation.

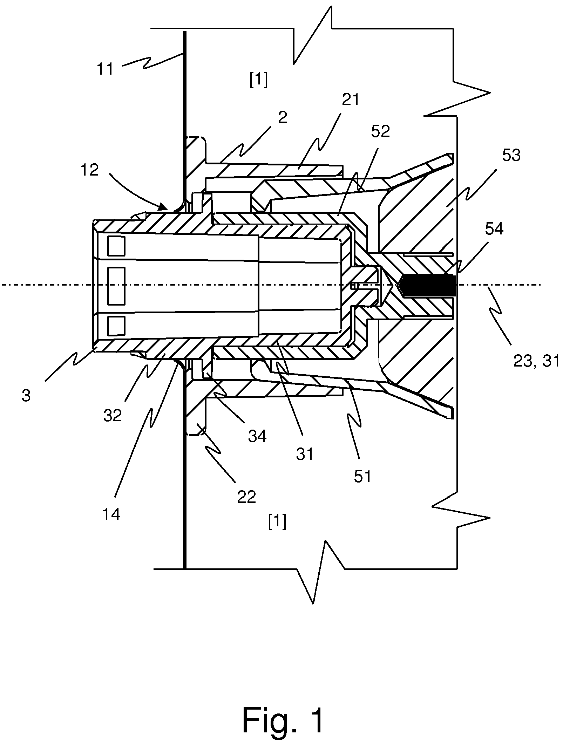

On s'intéresse dans un premier temps à la figure 1 qui permet de présenter les caractéristiques techniques générales, fonctionnelles et dimensionnelles, du dispositif de couplage selon l'invention. Comme l'illustre la figure 1 celui-ci comporte principalement:
- une enveloppe 1, destinée à renfermer l'outil de mise en rotation de telle façon que celui-ci puisse être utilisé dans un environnement stérile sans que son introduction dans cet environnement puisse contaminer ce dernier.
- un embout de liaison 3 assurant la liaison proprement dite entre l'extrémité d'un outil d'entrainement en rotation 5, logé à l'intérieur de l'enveloppe 1, et l'instrument chirurgical que ce dernier doit entrainer, placé à l'extérieur de l'enveloppe. A cet effet l'embout de liaison 3 est configuré et agencé au sein du dispositif de façon à traverser la paroi de l'enveloppe 1.

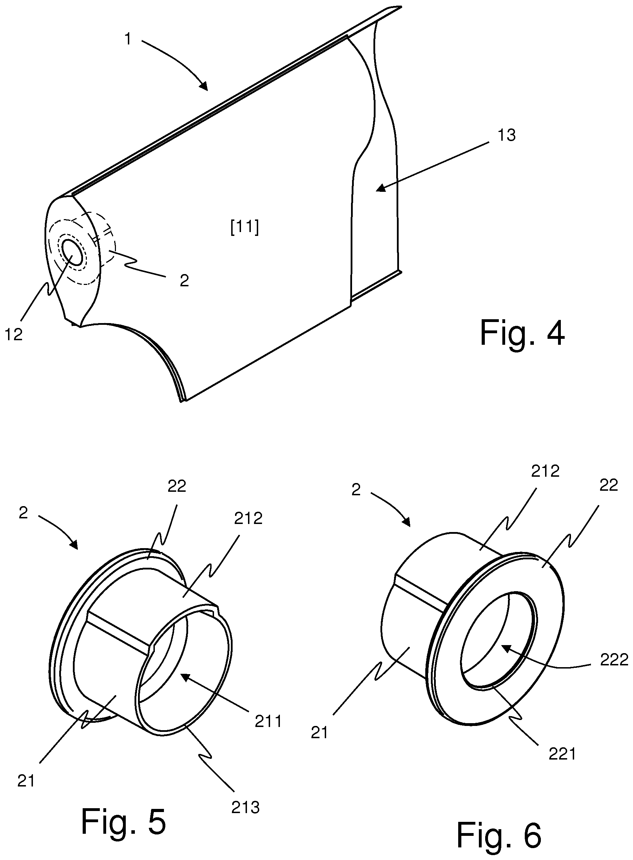

L'enveloppe 1 du dispositif de couplage selon l'invention, illustrée plus en détail par la vue schématique en perspective de la figure 4, comporte un sachet 11 en forme de poche, destiné à servir de logement à l'outil d'entrainement et comportant sur une de ses parois un orifice circulaire 12 (qui apparait traversée par l'élément 3 sur la figure 1).

Le sachet 11 comporte également une seconde ouverture 13 (non représentée sur la figure 1), dimensionnée de façon à permettre l'insertion facile d'un outil d'entrainement à l'intérieur du sachet, cette ouverture étant pourvu d'un moyen de fermeture permettant de refermer ladite ouverture de manière étanche.

L'enveloppe 1 comporte également une bague 2, qui comporte elle-même une partie tubulaire 21 formant un conduit 211. La bague 2 est configurée de façon à pouvoir se fixer, de manière amovible, sur l'extrémité 51 du corps 53 de l'outil d'entrainement 5 de telle façon que le montage rende la bague 2 et le corps 53 de l'outil d'entrainement 5 solidaires l'un de l'autre. La fixation de la bague 2 sur le corps de l'outil d'entrainement peut être réalisée par tout moyen approprié pouvant être placé, par exemple, à l'intérieur de la bague.

Selon l'invention la bague 2 est fixée sur la paroi interne du sachet 11 par une de ses extrémités, au niveau de l'ouverture 12, de telle façon que le centre de l'ouverture 12 soit aligné sur l'axe de symétrie 23 de la partie tubulaire 21 de la bague. En particulier, la bague tubulaire 2 est positionnée sur la surface interne de l'enveloppe 1 et solidaire de cette dernière par une de ses extrémités 22 de sorte que l'ensemble de la bague tubulaire 2 est disposée à l'intérieur de l'enveloppe 1 lorsque l'outil d'entrainement se trouve dans l'enveloppe 1. En d'autres termes, aucune portion ou partie de la bague tubulaire 2 n'est disposée à l'extérieur de l'enveloppe 1 lorsque l'outil d'entrainement se trouve dans l'enveloppe 1. Cette position interne de la bague tubulaire 2 permet notamment d'obtenir un encombrement extérieur à l'enveloppe 1 minimal notamment par rapport à une bague tubulaire comprenant une portion extérieure, tel que dans le document WO 2015/154188 décrit ci-avant.

De plus, la bague tubulaire 2 peut être formée de manière monobloc, c'est-à-dire d'une seule pièce. Ainsi, l'assemblage de la bague tubulaire 2 à l'enveloppe 1 est rendu aisé par rapport à une bague comprenant plusieurs pièces à assembler. En effet, dans ce premier cas, une seule pièce est à manipuler pour solidariser la bague tubulaire 2 à l'enveloppe 1.

L'embout de liaison 3, est un élément de forme allongée présentant une symétrie de révolution autour de son axe longitudinal 31 (confondu avec l'axe 23 sur la figure 1). Lors de l'assemblage des différents éléments du dispositif de couplage selon l'invention, il est positionné de façon à traverser la paroi du sachet 11 par l'ouverture 12, en passant à l'intérieur de la bague 2.

L'embout de liaison 3 comporte un tronçon distal 32 cylindrique dont une partie 321, de section circulaire, est destinée à être placée, au moins partiellement, à l'extérieur de l'enveloppe 11 lorsque l'embout de liaison 3 est mis en place, ainsi qu'un tronçon proximal 31 destiné à venir se loger à l'intérieur de l'enveloppe 1.

Ainsi, selon l'invention, l'extrémité du tronçon distal 32 est configurée de façon à constituer une interface mécanique entre l'embout de liaison 3 et l'extrémité d'un instrument chirurgical, tandis que l'extrémité du tronçon proximal 31 est configurée de façon à constituer une interface mécanique entre l'embout de liaison 3 et l'embout de couplage 52 de l'outil d'entrainement 5. Selon la configuration d'interface adoptée, l'extrémité du tronçon proximal 31 est configurée pour venir se fixer sur ou dans l'embout de couplage 52 équipant l'outil d'entrainement en rotation 5 considéré.

Selon l'invention également, ces interfaces sont équipées de moyens de blocage permettant, durant l'opération réalisée et en l'absence d'action du praticien, le maintien des liaisons mécaniques entre l'embout de liaison et l'instrument chirurgical d'une part et de l'embout de liaison et de l'outil d'entrainement d'autre part.

Selon l'invention également, elles sont aussi pourvues de moyens permettant de maintenir colinéaires les axes de symétrie de l'instrument chirurgical percutané, de l'embout de liaison du dispositif selon l'invention et de l'embout de couplage de l'outil d'entrainement en rotation.

L'embout de liaison 3, est configuré et dimensionné, compte tenu des dimensions internes de la bague 2, de façon à pouvoir tourner librement à l'intérieur de la bague 2.

Il comporte en outre un moyen de blocage en translation 34 configuré pour coopérer avec la bague 2, de façon à limiter son mouvement de translation à l'intérieur de la bague de telle sorte qu'il ne puisse pas sortir totalement du sachet 11 par l'ouverture 12.

Dans une forme préférée de l'invention, le moyen de blocage 34 est également configuré et agencé de façon à maintenir l'embout de liaison dans une position pour laquelle son axe de symétrie 31 est sensiblement confondu avec l'axe de symétrie 23 de la partie tubulaire 21 de la bague 2, lorsque l'embout 3 est en place dans la bague 2.

Le diamètre du tronçon distal 32 est quant à lui défini de façon à être légèrement supérieur au diamètre de l'ouverture 12 pratiquée dans la paroi de l'enveloppe 1, de sorte que l'insertion de l'extrémité du tronçon distal de l'embout de liaison dans l'ouverture 12 entraine une déformation élastique de cette dernière formant un repli 14 autour de la paroi externe de la partie de section circulaire 321 du tronçon distal 32. Ce repli constitue avantageusement un joint d'étanchéité disposé au niveau de la première ouverture 12 de l'enveloppe. Ainsi, l'étanchéité entre l'extérieur et l'intérieur de l'enveloppe 1 est réalisée par l'enveloppe 1 elle-même. Ceci permet au dispositif de couplage de s'affranchir de l'ajout d'un joint d'étanchéité rapporté et de la réalisation d'étapes de fabrication permettant le montage de celui-ci. Une telle étanchéité réalisée directement par l'enveloppe 1 permet une réduction de la difficulté de fabrication et d'assemblage notamment par rapport à un système dans lequel l'étanchéité est réalisée par un joint d'étanchéité rapporté et monté à l'intérieur de gorges usinées.

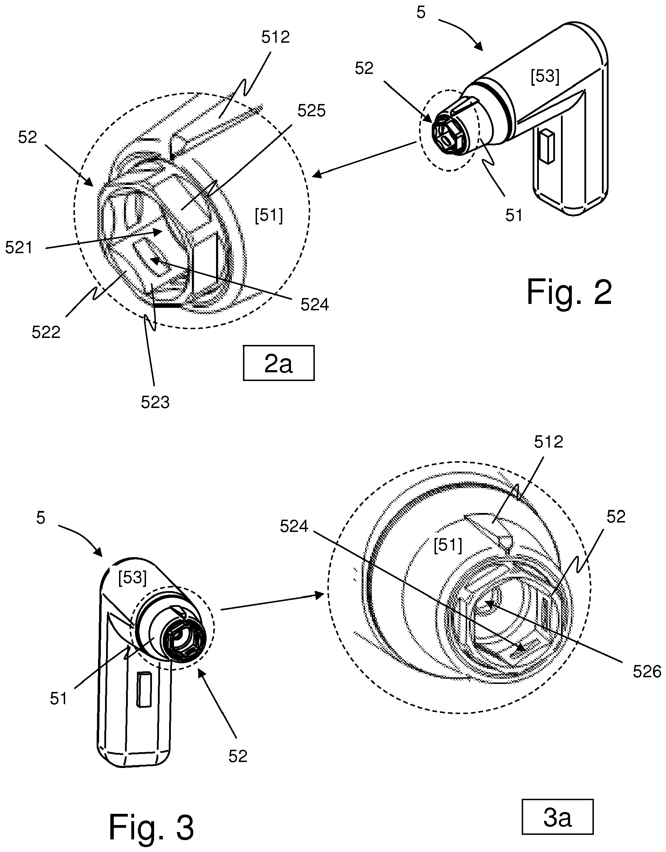

Les figures 2 à 10 permettent de présenter de manière plus détaillée, outre les caractéristiques techniques principales de l'invention, des caractéristiques techniques additionnelles, en rapport notamment, mais non exclusivement, avec un mode de réalisation pris comme exemple.

Ce mode de réalisation est notamment adapté au couplage stérile d'un instrument de biopsie avec d'outil d'entrainement en rotation particulier présentant un embout de couplage tel que celui illustré par les figures 2 et 3.

L'exemple d'outil d'entrainement 5, illustré par les figures 2-2a et 3-3a, comporte un corps 53, représentée schématiquement, qui présente une extrémité 51 de forme conique de laquelle émerge un embout de couplage 52 monté libre en rotation par rapport au corps 53 de l'outil 5.

Dans un mode de réalisation préféré, correspondant à l'exemple de réalisation présenté, l'extrémité 51 du corps 53 de l'outil d'entrainement présente sur sa paroi externe une clavette longitudinale 512.

De manière générale, l'embout de couplage 52 est quant à lui configuré de façon à pouvoir être lié mécaniquement à l'embout de liaison 3 du dispositif de couplage selon l'invention.

Cet embout de couplage 52, entrainé en rotation, par l'arbre moteur 54 (voir figure 1) de l'outil d'entrainement 5 sur lequel il est monté, lorsque l'outil d'entrainement est mis en marche, est configuré de telle façon qu'il constitue une interface mécanique avec l'extrémité de l'instrument chirurgical qu'il est destiné à entrainer en rotation.

Dans l'exemple de réalisation considéré ici, l'embout de couplage 52 se présente comme une cavité 521 comportant une paroi interne qui présente une zone arrière 523 de forme hexagonale et une extrémité avant 522 de forme tronconique.

Par ailleurs, une gorge chanfreinée 524 est réalisée dans la paroi interne de la cavité 521 au niveau de la zone arrière hexagonale 523.

L'embout de couplage 52 comporte également un perçage cylindrique borgne 526 formant le fond de l'embout, dont l'axe de symétrie est confondu avec l'axe de symétrie général de l'embout de couplage 52.

Il comporte en outre une paroi externe 525 de section hexagonale dont les pans sont disposés en regard des pans formant la zone arrière hexagonale 523 de la paroi interne de l'embout de couplage 52. Une telle disposition sert avantageusement de repère visuel afin d'orienter correctement l'embout de couplage 52 pour faciliter l'insertion du moyen d'entrainement 5 sur l'extrémité de l'embout de liaison 3.

La figure 4 présente une vue schématique en perspective de l'enveloppe 1 équipée de sa bague 2 selon l'invention.

Comme décrit précédemment, l'enveloppe 1 comporte un sachet 11 en forme de poche, destinée à servir de logement à l'outil d'entrainement 5, sur la paroi duquel est fixée la bague 2 (représentée en traits pointillés) en regard de l'ouverture 12.

Il est à noter que, de manière préférentielle, l'ouverture 12 est positionnée sur la paroi du sachet 11, comme illustré par la figure 4, de façon à faciliter l'insertion de l'extrémité 51 de l'outil d'entrainement 5 dans la partie tubulaire 21 de la bague 2 lorsque l'on introduit ledit outil dans le sachet 11.

Comme l'illustrent les figures 5 et 6, la bague 2 comporte, dans cet exemple de réalisation, un corps tubulaire 21 et un flasque d'extrémité 22, le corps tubulaire 21 formant un conduit interne 211 dans laquelle l'extrémité 51 du corps de l'outil d'entrainement en rotation 5 vient s'emboiter de telle façon que la bague 2 et l'extrémité 51 du corps de l'outil 5 sont mécaniquement liés l'un de l'autre.

Le flasque 22, en forme de disque, est placé à l'extrémité du corps tubulaire 21 de la bague 2 en contact avec la paroi de l'enveloppe 1. Il présente une ouverture centrale 222, de préférence circulaire, dont le centre est situé sur l'axe de symétrie 23 du corps tubulaire 21.

Par suite, la bague 2 est fixée sur la paroi interne du sachet 11 par son flasque d'extrémité 22, au niveau de l'ouverture 12, de telle façon que le centre de l'ouverture 12 soit aligné sur l'axe de symétrie du corps tubulaire 21 de la bague

De manière préférentielle, la section de l'ouverture 222 est plus petite que la section du conduit interne 211, de sorte que la paroi du flasque forme un collet interne qui constitue une butée circulaire à l'extrémité du corps tubulaire 21.

Dans le présent exemple de réalisation, le conduit 211 présente une forme conique coopérant avec la forme conique de l'extrémité 51 du corps 53 de l'outil d'entrainement en rotation 5, représenté sur les figures 2-2a et 3-3a.

Les conicités des deux formes coniques sont ici définies de façon à ce que, lors de l'insertion de l'extrémité 51 du corps de l'outil d'entrainement 5 dans le conduit 211, les deux éléments 21 et 51 se coincent l'un dans l'autre ce qui tend à maintenir la bague 2, serrée sur l'extrémité 51 du corps de l'outil d'entrainement 5, en position fixe par rapport à ce dernier.

Dans le mode de réalisation décrit ici, le corps tubulaire 21 est en outre configuré pour permettre l'introduction dans le conduit 211 de la clavette longitudinale 512 placée sur la surface externe de l'extrémité 51 du corps de l'outil d'entrainement 5. A cet effet la paroi du conduit 211 présente une échancrure longitudinale 212 destinée à accueillir la clavette 512 lors de l'insertion de l'extrémité 51 du corps de l'outil d'entrainement dans le conduit 211.

Avantageusement la clavette 512 insérée dans l'échancrure 212 du conduit 211 interdit tout mouvement de rotation de la bague 2 relativement à l'extrémité 51 du corps de l'outil d'entrainement 5. On évite ainsi, de manière avantageuse, tout risque de déformation en torsion de l'enveloppe 1, entrainée par une éventuelle rotation intempestive de la bague 2 qui pourrait compromettre l'étanchéité de l'enveloppe, cette rotation pouvant être induite par le frottement du corps de l'embout de liaison 3 contre le repli 14 de l'ouverture 12 de l'enveloppe 1, lorsque l'embout de liaison 3 est entrainé en rotation par l'embout de couplage 52 de l'outil d'entrainement 5.

D'un point de vue réalisation la bague 2 est fixée sur la paroi interne du sachet 11 par son flasque d'extrémité 22 dont la face distale est fixée, par soudage ou collage, sur la paroi interne du sachet le sachet 11.

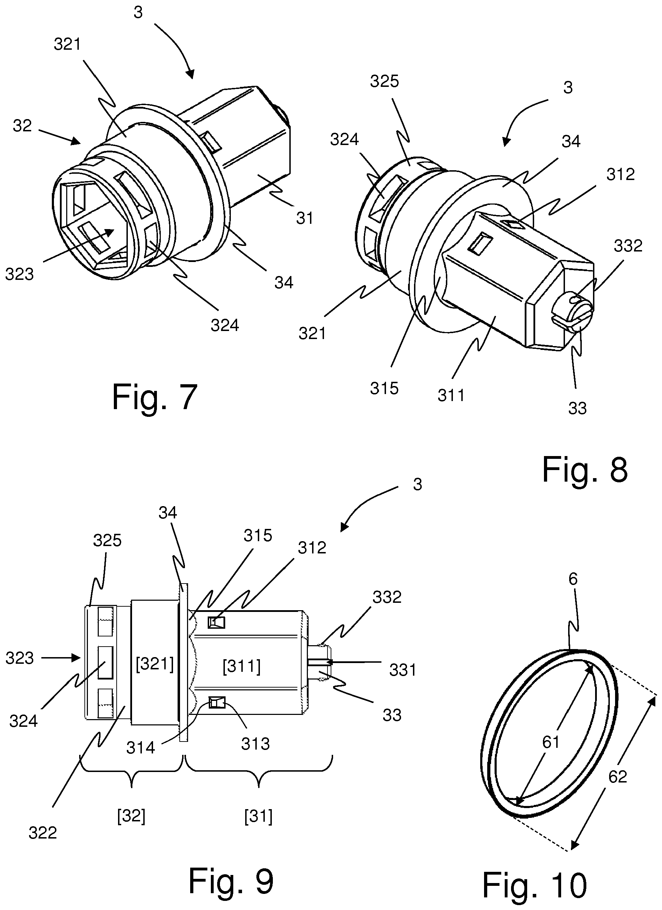

Comme cela a été décrit précédemment, l'embout de liaison 3 comporte, comme l'illustrent les figures 7 à 9, un tronçon distal tubulaire 32 prolongé par un tronçon proximal 31.

Le tronçon distal tubulaire 32 comporte un corps cylindrique 321, de section externe circulaire, définissant une cavité interne formant une zone d'accueil 323 (cavité) destinée à recevoir l'extrémité de l'instrument chirurgical percutané auquel on veut accoupler l'outil d'entrainement 5.

Selon l'invention, la longueur du tronçon distal 32 est principalement définie de telle façon que, lorsque l'embout de liaison 3 est mis en place, l'extrémité libre 325 du tronçon 32 est située à l'extérieur de l'enveloppe 1.

Le diamètre externe du corps cylindrique 321 est quant à lui défini de façon à être légèrement supérieur au diamètre de l'ouverture 12 pratiquée dans la paroi de l'enveloppe 1, de sorte que l'insertion de l'extrémité libre du tronçon distal 32 de l'embout de liaison 3 dans l'ouverture 12 entraine une déformation élastique de cette dernière formant un repli 14 autour de la paroi externe du corps cylindrique 321, repli visible sur la figure 1. Ce repli contribue à assurer l'étanchéité de l'enveloppe 1 au niveau de l'ouverture 12 en isolant la zone non stérile délimitée par l'enveloppe 1 et l'espace extérieur réputé stérile.

Dans la forme de réalisation prise comme exemple, la cavité 323 est une cavité de section hexagonale sur les parois de laquelle sont ménagées, au niveau de l'extrémité 325, des ouvertures 324 permettant l'emboitage d'ergots de fixation placés à l'extrémité de l'instrument chirurgical percutané. L'embout d'extrémité de l'instrument chirurgical percutané peut alors, par exemple, prendre la forme d'un cylindre à base hexagonale, destiné à venir se loger dans une cavité cylindrique aux parois hexagonales, ménagée dans un moyen de mise en rotation donné.

La longueur de l'embout d'extrémité de l'instrument, la profondeur de la cavité, ainsi que les dimensions générales des tronçons de l'embout et de la cavité sont principalement définies de telle façon que l'instrument de biopsie et le moyen de mise en rotation soient assemblés l'un à l'autre sans jeu, de sorte que la mise en œuvre par le praticien dudit moyen, poignée ou outil à embout rotatif, entraine une rotation de l'instrument sur lui-même autour de son axe de symétrie et une pénétration de l'instrument selon une direction sensiblement rectiligne.

Le tronçon proximal 31 est quant à lui configuré et dimensionné pour réaliser la liaison, avec un minimum de jeu, entre l'embout de liaison 3 et l'embout de couplage 52 situé à l'extrémité du corps de l'outil d'entrainement 5. La liaison avec l'embout de couplage 52 est réalisée par la paroi externe du tronçon proximal.

Selon l'invention, le tronçon proximal 31 définit par ailleurs une cavité interne qui prolonge la cavité 323 du tronçon distal 32, comme l'illustre la figure 1, de telle façon que l'embout de l'instrument chirurgical percutané puisse se loger totalement dans l'embout de liaison 3. Cette cavité est fermée du côté de l'extrémité du tronçon proximal destiné à s'interfacer avec l'embout de couplage 52 de l'outil d'entrainement 5.

Dans l'exemple de réalisation considéré, la paroi externe du tronçon proximal 31 forme un élément mâle de section hexagonale, dimensionné de façon à être inséré, avec un minimum de jeu, dans l'empreinte hexagonale 523 de l'embout de couplage 52 illustrée par les figures 2a et 3a.

Sa paroi externe comporte également une zone tronconique 315 conformée et agencée sur la paroi de façon coopérer avec l'extrémité avant 522 de forme tronconique de l'embout de couplage 52 de l'outil d'entrainement 5, lorsque l'embout de couplage est en place sur l'embout de liaison 3. La zone tronconique 315 vient alors s'emboiter dans l'extrémité avant 522 de l'embout de couplage 52 de façon à assurer le maintien sans jeu de la coaxialité des deux éléments 3 et 52 lors de l'utilisation.

Dans l'exemple de réalisation décrit ici le corps 311 du tronçon proximal 31 comporte également, au niveau de son extrémité, un élément d'extrémité cylindrique 33, fendu sur sa longueur, destiné à être inséré dans le perçage cylindrique borgne 526 formant le fond de l'embout de couplage 52 de l'outil d'entrainement 5.

De manière préférentielle le diamètre du perçage 526 est légèrement supérieur au diamètre de la section de l'élément d'extrémité 33.

Cependant, la paroi externe de l'élément d'extrémité 33 comporte par ailleurs des bossages 332 formant des surépaisseurs telles que le diamètre de la section de l'élément d'extrémité est localement supérieur au diamètre du perçage 526. Néanmoins, la présence de la fente 331 permet de rendre l'élément d'extrémité 33 déformable de telle façon que lors du couplage de l'élément de liaison 3 à l'embout de couplage 52 de l'outil d'entrainement 5, les bossages 332 frottent contre la paroi du perçage 526 ce qui permet avantageusement d'éviter que l'embout de liaison 3 ne bouge librement dans l'embout de couplage 52.

Par ailleurs, le corps 311 du tronçon proximal 31 comporte, sur sa paroi externe, des bossages chanfreinés 312 conformés pour pouvoir se loger dans la gorge chanfreinée 524 réalisée dans la paroi interne de la cavité 521 au niveau de la zone arrière hexagonale 523 de l'embout de couplage 52 et assurer le blocage par encliquetage du corps 311 du tronçon proximal 31 de l'embout de liaison 3 dans l'embout de couplage 52.

Selon l'invention, les bossages chanfreinés 312 comportent des chanfreins 313 et 314 qui peuvent être identiques ou différents.

La valeur des angles d'inclinaison de ces chanfreins permet de régler la force d'insertion ou d'extraction de l'embout de liaison 3 dans ou hors de l'embout de couplage 52. Cette force, d'environ 2kg, est définie par la hauteur des bossages chanfreinés 312 par rapport à la dimension de l'empreinte hexagonale 523 de l'embout de couplage 52.

Ces bossages chanfreinés 312 peuvent être par exemple au nombre de quatre, repartis symétriquement autour de la paroi du corps 311 qui se logent dans la gorge chanfreinée 524 de l'embout de couplage 52.

Selon l'invention l'embout de liaison 3 comporte également, comme cela a été dit précédemment, un moyen de blocage, configuré pour coopérer avec la bague 2, de façon à limiter le mouvement de translation de l'embout de liaison 3 à l'intérieur de la bague 2.

Dans le présent exemple de réalisation, le moyen de blocage consiste en une protubérance annulaire 34, disposée sur la surface externe de l'embout et placée dans un plan perpendiculaire à l'axe longitudinal de l'embout de liaison 3, à la limite entre le tronçon distal 32 et le tronçon proximal 31. Cette protubérance est dimensionnée de façon à ce que, lorsque l'embout de liaison 3 se déplace dans la bague 2 de l'intérieur vers l'extérieur du sachet 11, l'embout vient en fin de course buter sur la paroi du flasque 22 de la bague 2.

La protubérance annulaire 34 permet par ailleurs d'éviter que des particules ne se propagent sur le tronçon distal 32 de l'embout de liaison 3 pendant son insertion dans l'embout de couplage 52.

Elle contribue en outre à constituer un système de chicanes au niveau de l'ouverture 12 de l'enveloppe 1 qui réduit le risque que des particules ne sortent de l'enveloppe 1 lorsque le dispositif est assemblé.

Elle assure également que la bague 2 et l'embout de liaison 3 soient bien placés coaxialement, de façon à ce que la déformation de l'ouverture 12 de l'enveloppe 11 soit uniformément répartie autour du corps cylindrique 321 de l'élément de liaison 3.

Dans une forme particulière de réalisation, le dispositif selon l'invention peut comporter une bague repère 6 telle que celle illustrée par la figure 10.

La bague repère 6, d'une couleur différente des autres éléments du dispositif, s'encliquette dans une gorge 322 localisée au niveau du corps cylindrique 321 du tronçon distal 32 de l'embout de liaison 3.

Le diamètre intérieur 61 de la bague 6 est supérieur ou égale au diamètre de la gorge 322 tout en étant inférieur au diamètre externe du corps cylindrique 321. Le diamètre extérieur 62 est, quant à lui, supérieur au diamètre externe du corps cylindrique 321, de façon à assurer une rétention du sachet 1 au niveau de l'ouverture 12.

La largeur de la bague repère 6 est par ailleurs inférieure à la largeur de la gorge 322.

La différence de couleur permet au praticien de constater d'un coup d'œil que la bague repère 6 est bien visible et ainsi de vérifier que le sachet occupe une position conforme dans la zone de la première ouverture 12 de l'enveloppe, position garantissant une bonne étanchéité du dispositif.

Comme tend à le montrer la description qui précède, la structure du dispositif selon l'invention permet ainsi avantageusement, en particulier dans la forme de réalisation préférée prise comme exemple, de garantir une étanchéité entre la zone de l'espace limitée par l'enveloppe 1 et susceptible de renfermer un matériel non stérile (l'outil d'entrainement en particulier) et le reste de l'espace dans lequel le dispositif est mise en œuvre, espace par principe stérile.

Compte tenu des caractéristiques techniques du dispositif de couplage selon l'invention, il est ainsi avantageusement possible de réaliser de manière simple le couplage amovible d'un outil d'entrainement 5, outil non stérile, à un instrument de biopsie nécessairement stérile, sans risquer de contaminer de manière involontaire le milieu stérile durant l'opération de couplage ou celle inverse de séparation.

Il est à considérer ici que compte tenu de la relation étroite existant nécessairement entre les structures des extrémités respectives de l'embout de liaison 3 du dispositif selon l'invention et de l'embout de couplage 52 de l'outil d'entrainement en rotation 5, destinées à coopérer pour assurer la liaison de ces deux éléments, l'invention porte non seulement sur un dispositif de couplage présentant des caractéristiques techniques et morphologiques le rendant capable de réaliser de manière simple le couplage amovible d'un outil d'entrainement 5, outil non stérile, à un instrument de biopsie nécessairement stérile en garantissant le confinement de l'outil d'entrainement, mais également sur un ensemble permettant d'assurer de manière optimale la mise en rotation de l'instrument de biopsie par l'outil d'entrainement. Par manière optimale on entend ici une manière permettant notamment de limiter les jeux pouvant exister au niveau des différentes interfaces formant la chaine mécanique reliant l'outil d'entrainement et l'instrument de biopsie.

L'invention concerne donc aussi, naturellement, un système de couplage stérile entre un instrument de biopsie et un outil de mise en rotation dudit instrument, le système comportant le dispositif de couplage exposé précédemment ainsi que l'embout de couplage 52 de l'instrument de biopsie à un moyen 5 d'entrainement en rotation, tel que décrit dans la présente description, embout permettant de monter sur l'instrument, de manière amovible, différents moyens permettant au praticien de mettre l'instrument en rotation, et que l'embout de couplage de l'outil d'entrainement en rotation à un instrument de biopsie tel que décrit également dans la présente description.

D'un point de vue opérationnel, l'opération de couplage stérile d'un instrument de biopsie et d'un outil d'entrainement dudit instrument au moyen d'un dispositif de couplage selon l'invention peut être décrite par les étapes d'implémentation suivantes :
- Une étape préliminaire de retournement de l'enveloppe 1 sur elle-même, cette étape étant réalisée en repoussant la bague 2 à l'intérieur de l'enveloppe 1 jusqu'à ce que son extrémité libre 213 émerge de l'enveloppe au travers de la seconde ouverture 13 de l'enveloppe 1 ;
- une première étape durant laquelle l'assistant enfonce l'embout de couplage 52 de l'outil d'entrainement 5 sur l'extrémité proximale 31 de l'embout de liaison 3, jusqu'à réaliser l'emboitement des deux éléments l'un dans l'autre, l'embout de liaison, stérile, étant disposé de telle façon que l'assistant n'ait pas à entrer en contact avec celui-ci ;
- une deuxième étape durant laquelle le praticien, équipé de gants stériles, saisit et présente l'enveloppe 1 retournée et durant laquelle l'assistant, après avoir introduit l'extrémité distale 51 de l'outil d'entrainement 5, dans laquelle est insérée l'extrémité de l'embout de liaison 3, dans la bague 2 de l'enveloppe 1, lâche l'outil d'entrainement 5, celui-ci étant alors maintenu par le praticien au moyen de l'enveloppe 1 retournée sur elle-même.

A ce moment, l'extrémité du tronçon cylindrique distal 32 de l'embout de liaison 3 passe au travers de l'ouverture cylindrique 12 de l'enveloppe 1 provoquant sa déformation élastique et assurant l'étanchéité de l'enveloppe au niveau de l'ouverture 12.

Dans le même temps, du fait du poids de l'outil d'entrainement l'enveloppe 1 reprend partiellement sa forme dépliée.
- une troisième étape durant laquelle le praticien termine de remettre en forme l'enveloppe 1 sans toucher à l'outil d'entrainement 5, de façon à ce que l'enveloppe 1 enferme l'outil d'entrainement 5, puis referme hermétiquement la seconde ouverture 13 de l'enveloppe 1 à l'aide de moyens de fermeture disposés au niveau de ladite ouverture ;
- une quatrième étape durant laquelle le praticien met en place l'instrument chirurgical percutané en insérant l'embout de liaison dudit instrument dans l'extrémité 323 du tronçon distal 32 de l'embout de liaison 3.

Selon l'invention, l'étape préliminaire peut être soit implémentée de manière indépendante au moment de la fabrication du dispositif de couplage selon l'invention, le dispositif est alors livré au praticien sous emballage stérile avec son enveloppe 1 pré-retournée; soit implémentée par le praticien juste avant son utilisation, le dispositif étant alors livré sous emballage stérile avec son enveloppe 1 non retournée sur elle-même.

Considérant en particulier le système de couplage présenté à titre d'exemple de réalisation dans la description qui précède, la première phase est, dans ce cas, réalisée par l'assistant qui saisit l'outil d'entrainement 3 puis, après l'avoir orienté au moyen du repère visuel hexagonal 525, le glisse sur l'extrémité du tronçon distal 31 de l'embout de liaison 3, ledit embout de liaison étant par exemple maintenu verticalement dans une coque d'emballage (i.e. blister) médical, jusqu'à l'encliquetage des deux éléments.

La troisième étape est par exemple réalisée par le praticien lui-même qui saisit une extrémité de l'enveloppe 1 puis la soulève en faisant glisser la paroi externe repliée de l'enveloppe 1 de façon à finir d'envelopper l'outil d'entrainement 5, en prenant garde cependant que ses doigts n'entrent pas en contact avec le corps 53 de l'outil d'entrainement jusqu'à ce que l'enveloppe 1 reprenne sa forme initiale.

Ensuite, le praticien referme la seconde ouverture 13 de l'enveloppe 1 à l'aide des moyens de fermeture disposés au niveau de ladite ouverture et vérifie que la bague repère 6 est bien apparente, faute de quoi l'assemblage réalisé serait considéré comme défectueux.

Considérant en particulier le système de couplage présenté à titre d'exemple de réalisation dans la description qui précède, la dernière phase du processus de couplage, consiste pour le praticien à saisir l'ensemble constitué par l'outil d'entrainement 3 recouvert de l'enveloppe 1 et à insérer l'embout hexagonal de liaison de l'instrument de biopsie dans l'empreinte hexagonale 323 située à l'extrémité de la tronçon distale 32 de l'embout de liaison 3.

A l'issue de la dernière phase, le praticien dispose d'un outil de mise en rotation stérile qu'il peut à volonté coupler à un instrument de biopsie, puis découpler dudit instrument pour lui substituer une simple poignée avec laquelle il peut mettre l'instrument de biopsie en rotation sans assistance mécanique.

## Revendications

1. Dispositif de couplage stérile d'un instrument chirurgical percutané à un outil d'entrainement en rotation (5) dudit instrument, ledit outil (5) comportant une extrémité (51) munie d'un embout de couplage (52), comportant :
- une enveloppe (1) souple présentant une première ouverture (12) de forme circulaire et une seconde ouverture (13) permettant d'introduire l'outil d'entrainement (5) dans ladite enveloppe (1), la première ouverture (12) étant positionnée sur la paroi de l'enveloppe (1) de telle façon que, lorsque l'outil d'entrainement se trouve dans la poche, l'embout de couplage (52) se trouve placé en regard de ladite première ouverture (12);
- un embout de liaison (3), comportant un tronçon distal (32) cylindrique assurant le couplage avec l'instrument chirurgical percutané et un tronçon proximal (31) assurant le couplage avec l'outil d'entrainement (5), configuré pour traverser l'enveloppe (1) par la première ouverture circulaire (12) de telle sorte que l'extrémité (323) de son tronçon distal (32) soit placée à l'extérieur de l'enveloppe (1), son tronçon proximal (31) étant placé à l'intérieur de l'enveloppe (1);
- une bague tubulaire (2) formant un conduit (211) dans laquelle l'embout de liaison est destiné à être inséré, positionnée sur la surface interne de l'enveloppe (1) en regard de la première ouverture circulaire (12), ladite bague (2) étant solidaire de l'enveloppe (1) par une de ses extrémités (22), son autre extrémité (213) J Z étant configurée pour solidariser l'enveloppe à la paroi de l'outil d'entrainement (5) par insertion de l'extrémité (51) du corps de l'outil d'entraînement (5) dans le conduit (211), ledit conduit (211) étant configuré de telle façon que l'embout de liaison (3) puisse tourner librement autour de son axe à l'intérieur de la bague (2) tout en étant maintenu axialement;
l'enveloppe (1), la bague (2) et l'embout de liaison (3) comportant des moyens qui coopèrent pour former une barrière étanche au niveau de la première ouverture circulaire (12), ladite barrière étant configurée de façon à ne pas entraver la rotation de l'embout de liaison (3).

2. Dispositif selon la revendication 1, **caractérisé en ce que** le tronçon distal (32) de l'embout de liaison (3) présente une forme tubulaire et comporte une empreinte (323) configurée pour accueillir l'extrémité proximale d'un instrument chirurgical percutané, l'extrémité du tronçon proximal (31) de l'embout de liaison (3) étant configurée pour pouvoir se loger dans l'embout de couplage (52) de l'outil d'entrainement (5).

3. Dispositif selon la revendication 1 ou 2, **caractérisé en ce que** la bague (2) comporte un flasque (22) en forme de disque situé à l'extrémité de la partie tubulaire (21) par laquelle ladite bague est fixée sur la paroi interne de l'enveloppe (1), le flasque présentant une ouverture centrale (222) dont le centre est situé sur l'axe de symétrie de la partie tubulaire (21), la bague et le flasque étant fixés sur la paroi interne de l'enveloppe (1) de telle façon que le centre de la première ouverture (12) soit également aligné sur l'axe de symétrie de la partie tubulaire (21).

4. Dispositif selon la revendication 3, **caractérisé en ce que** l'embout de liaison (3) comporte en outre une protubérance annulaire (34) positionnée sur sa surface externe et délimitant le tronçon distal (32) et le tronçon proximal (31); le diamètre extérieur de la protubérance annulaire (34) étant sensiblement égale, à un jeu fonctionnel près, au diamètre intérieur du corps tubulaire (21) de la bague (2) et supérieur au diamètre de l'ouverture (222) du flasque (22), de telle sorte que l'embout de liaison (3) puisse coulisser dans la bague (2) suivant un axe sensiblement confondu avec l'axe de symétrie (23) du corps tubulaire (21) et traverser le flasque (22) jusqu'à ce que la protubérance annulaire (34) vienne en butée sur la paroi interne du flasque (22), l'axe de symétrie (33) de l'embout de liaison étant alors maintenu sensiblement confondu avec l'axe de symétrie (23) du corps tubulaire (21) par la protubérance annulaire (34), le flasque, la paroi intérieure du corps tubulaire (21) de la bague et la protubérance annulaire, formant une chicane limitant la possibilité de passage d'éléments fluides ou solides entre l'intérieur et l'extérieur de l'enveloppe par la première ouverture (12).

5. Dispositif selon la revendication 4, **caractérisé en ce que** la première ouverture (12) de l'enveloppe souple (1) présente un diamètre légèrement inférieur au diamètre externe du tronçon distal cylindrique (321) de l'embout de liaison (3) ledit diamètre étant défini de telle sorte que lorsque l'embout de liaison est inséré dans cette ouverture (12) depuis l'intérieur de l'enveloppe (1), le bord de ladite première ouverture (12) se déforme et constitue un repli annulaire (14) dirigé vers l'extérieur de l'enveloppe (1) qui vient entourer la surface externe du corps cylindrique (321) du tronçon distal (32) de l'embout (3), ce repli constituant un joint qui coopère avec la chicane formée par le flasque (22) de la bague (2), la paroi intérieure du corps tubulaire (21) de la bague (2) et la protubérance annulaire (34) de l'embout de liaison (3) pour constituer une barrière étanche au niveau de la première ouverture (12) de l'enveloppe (1).

6. Dispositif selon l'une quelconque des revendications 1 à 5, **caractérisé en ce que**, le corps de l'outil d'entrainement (5) présentant une forme conique au niveau de l'extrémité (51) munie de l'embout de couplage (52), l'extrémité de la bague tubulaire (2) présente une ouverture (211) de forme conique dimensionnée de telle façon que lorsque l'extrémité (51) de l'outil d'entrainement est inséré dans la bague (2), cette insertion provoque un emmanchement serré de la bague sur la l'extrémité (51) de l'outil d'entrainement (5) qui bloque tout mouvement relatif en translation et/ou en rotation de l'enveloppe (1) par rapport à l'outil d'entrainement (5).

7. Dispositif selon la revendication 6, **caractérisé en ce que** la paroi de l'extrémité (51) du corps de l'outil (5) comportant une protubérance longitudinale (512) formant une clavette, la paroi interne de la bague tubulaire (2) comporte une échancrure longitudinale (212) dimensionnée pour recevoir ladite clavette (512), l'ensemble clavette-échancrure formant un moyen complémentaire de blocage du mouvement relatif en en rotation de l'enveloppe (1) par rapport à l'outil d'entrainement (5).

8. Dispositif selon l'une quelconque des revendications précédentes, **caractérisé en ce que**, l'embout de couplage (52) de l'outil d'entrainement (5) formant une cavité (521) comportant une zone arrière (523) de forme hexagonale et une extrémité avant (522) de forme tronconique, le tronçon proximal (31) de l'embout de liaison (3) forme un élément mâle de section hexagonale, dimensionné de façon à pouvoir s'insérer, avec un minimum de jeu, dans la cavité de section hexagonale (521) de l'embout de couplage (52), la paroi externe dudit tronçon proximal (31) comportant une zone tronconique (315) agencée sur ladite paroi et conformée pour coopérer avec l'extrémité avant (522) de forme tronconique de l'embout de couplage (52), de façon à assurer le maintien de la coaxialité des deux éléments lors de l'utilisation.

9. Dispositif selon la revendication 8, **caractérisé en ce que**, la paroi interne de la cavité (521) de l'embout de couplage (52) de l'outil d'entrainement (5) comportant une gorge chanfreinée (524) au niveau de la zone arrière hexagonale (523), le corps (311) du tronçon proximal (31) de l'embout de liaison (3) comporte des bossages chanfreinés (312) conformés pour pouvoir se loger dans ladite gorge chanfreinée (524) et assurer un blocage par encliquetage du corps (311) du tronçon proximal (31) de l'embout de liaison (3) dans l'embout de couplage (52).

10. Dispositif selon l'une des revendications 8 ou 9, **caractérisé en ce que**, la cavité formée par l'embout de couplage (52) de l'outil d'entrainement (5) comportant un perçage cylindrique borgne (526) formant le fond de la cavité et dont l'axe de symétrie est confondu avec l'axe de symétrie général de l'embout de couplage (52), le corps (311) du tronçon proximal (31) de l'embout de liaison (3) comporte à son extrémité un élément d'extrémité cylindrique (33) fendu sur sa longueur, et dont le diamètre est défini de façon à ce que son insertion dans le perçage cylindrique borgne (526) se fasse avec un minimum de jeu.

11. Dispositif selon la revendication 10, **caractérisé en ce que** la paroi externe de l'élément d'extrémité (33) du corps (311) du tronçon proximal (31) de l'embout de liaison (3) comporte des bossages (332) formant des surépaisseurs telles que le diamètre de la section de l'élément d'extrémité (33) est localement supérieur au diamètre du perçage (526) de l'embout de couplage (52) de l'outil d'entrainement (5) et que l'emboitement de l'élément d'extrémité (33) du corps (311) du tronçon proximal (31) de l'embout de liaison s'effectue sans jeu.

12. Dispositif selon l'une des revendications précédentes, **caractérisé en ce que** l'extrémité distale (32) l'embout de liaison (3) comporte une empreinte hexagonale (323) permettant de recevoir l'extrémité proximale d'un instrument percutané chirurgical, cette extrémité proximale comportant un embout de section hexagonale.

13. Dispositif selon l'une des revendications précédentes, **caractérisé en ce que** le corps cylindrique (321) du tronçon distal (32) de l'embout de liaison (3) comporte une gorge annulaire (322) apte à recevoir une bague repère (6), ladite bague repère étant agencée sur le corps cylindrique (21) de telle façon que lorsque le couplage est effectué, si la barrière étanche au niveau de la première ouverture (12) de l'enveloppe (1) est effective, la bague repère est visible par le praticien.

14. Dispositif selon l'une des revendications précédentes, **caractérisé en ce que** la bague tubulaire (2) est formée de manière monobloc.

15. Dispositif selon l'une des revendications précédentes, **caractérisé en ce que** la bague tubulaire (2) est positionnée sur la surface interne de l'enveloppe (1) et solidaire de l'enveloppe (1) par une de ses extrémités (22) de sorte que l'ensemble de la bague tubulaire (2) est disposée à l'intérieur de l'enveloppe (1) lorsque l'outil d'entrainement se trouve dans l'enveloppe (1).

16. Procédé pour réaliser le couplage stérile d'un instrument de biopsie et d'un outil d'entrainement dudit instrument au moyen d'un dispositif de couplage selon l'une quelconque des revendications 1 à 15, comportant les étapes suivantes:
- une étape préliminaire de retournement de l'enveloppe (1) sur elle-même, cette étape étant réalisée en repoussant la bague (2) à l'intérieur de l'enveloppe (1) jusqu'à ce que son extrémité libre (213) émerge de l'enveloppe au travers de la seconde ouverture (13) de l'enveloppe (1);
- une première étape durant laquelle l'assistant assemble l'embout de couplage (52) de l'outil d'entrainement (5) sur l'extrémité proximale (31) de l'embout de liaison (3) jusqu'à réaliser l'emboitement des deux éléments l'un dans l'autre;
- une deuxième étape durant laquelle le praticien saisit et présente l'enveloppe (1) retournée et durant laquelle l'assistant, après avoir introduit l'extrémité distale (51) de l'outil d'entrainement (5) dans la bague (2) de l'enveloppe (1), lâche l'outil d'entrainement (5), celui-ci étant alors maintenu par le praticien au moyen de l'enveloppe retournée sur elle-même de telle sorte que, du fait du poids de l'outil d'entrainement, l'enveloppe (1) reprend partiellement sa forme dépliée;
- une troisième étape durant laquelle le praticien termine de remettre en forme l'enveloppe (1) sans toucher à l'outil d'entrainement (5), de façon à ce qu'elle enferme l'outil d'entrainement (5) puis referme hermétiquement la seconde ouverture (13) de l'enveloppe (1);
- une quatrième étape durant laquelle le praticien met en place l'instrument chirurgical percutané en insérant l'embout de liaison dudit instrument dans l'extrémité (323) de l'embout de liaison (3).

17. Procédé selon la revendication 16, **caractérisé en ce que** le dispositif de couplage étant livré au praticien sous emballage stérile avec son enveloppe (1) pré-retournée, l'étape préliminaire est implémentée, de manière indépendante, au moment du conditionnement du dispositif de couplage.

## Patentansprüche

1. Vorrichtung zur sterilen Kopplung eines perkutanen chirurgischen Instruments mit einem Drehwerkzeug (5) des Instruments, wobei das Werkzeug (5) ein Ende (51) aufweist, das mit einem Kopplungselement (52) ausgestattet ist, umfassend:
- eine flexible Ummantelung (1) mit einer ersten kreisförmigen Öffnung (12) und einer zweiten Öffnung (13), die die Einführung des Werkzeugs (5) in die Ummantelung (1) ermöglicht, wobei die erste Öffnung (12) derart an der Wand der Ummantelung (1) positioniert ist, dass, wenn sich das Werkzeug im Beutel befindet, das Kopplungselement (52) der ersten Öffnung (12) zugewandt ist;
- ein Verbindungsstück (3) mit einem distalen zylindrischen Abschnitt (32), der die Kopplung mit dem perkutanen chirurgischen Instrument ermöglicht, und einem proximalen Abschnitt (31), der die Kopplung mit dem Werkzeug (5) ermöglicht, das derart konfiguriert ist, dass es die Ummantelung (1) über die erste kreisförmige Öffnung (12) derart durchquert, dass das Ende (323) seines distalen Abschnitts (32) außerhalb der Ummantelung (1) angeordnet ist, wobei dessen proximaler Abschnitt (31) im Inneren der Ummantelung (1) angeordnet ist;
- einen rohrförmigen Ring (2), der einen Durchgang (211) bildet, in den das Verbindungsstück einzuführen ist, und auf der Innenfläche der Ummantelung (1), der ersten kreisförmigen Öffnung (12) gegenüberliegend angeordnet ist, wobei der Ring (2) mit der Ummantelung (1) an einem ihrer Enden (22) verbunden ist, wobei deren anderes Ende (213) derart konfiguriert ist, dass es die Ummwantelung mit der Wand des Werkzeugs (5) verbindet, indem das Ende (51) des Körpers des Werkzeugs (5) in den Durchgang (211) eingeführt wird, wobei der Durchgang (211) derart konfiguriert ist, dass sich das Verbindungsstück (3) um seine Achse im Inneren des Rings (2) frei drehen und dabei axial festgehalten werden kann;
wobei die Ummantelung (1), der Ring (2) und das Verbindungsstück (3) Mittel umfassen, die zusammenwirken, um eine dichte Barriere auf Höhe der ersten kreisförmigen Öffnung (12) zu bilden, wobei die Barriere derart konfiguriert ist, dass die Rotation des Verbindungsstücks (3) nicht verhindert wird.

2. Vorrichtung nach Anspruch 1, **dadurch gekennzeichnet, dass** der distale Abschnitt (32) des Verbindungsstücks (3) rohrförmig ist und eine Einprägung (323) umfasst, die zur Aufnahme des proximalen Endes eines perkutanen chirurgischen Instruments konfiguriert ist, wobei das Ende des proximalen Abschnitts (31) des Verbindungsstücks (3) derart konfiguriert ist, dass es im Kopplungselement (52) des Werkzeugs (5) untergebracht werden kann.

3. Vorrichtung nach Anspruch 1 oder 2, **dadurch gekennzeichnet, dass** der Ring (2) einen scheibenförmigen Flansch (22) umfasst, der am Ende des rohrförmigen Teils (21) angeordnet ist, durch das der Ring an der Innenwand der Ummantelung (1) befestigt ist, wobei der Flansch eine zentrale Öffnung (222) aufweist, dessen Mittelpunkt auf der Symmetrieachse des rohrförmigen Teils (21) angeordnet ist, wobei der Ring und der Flansch an der Innenwand der Ummwantelung (1) derart befestigt sind, dass der Mittelpunkt der ersten Öffnung (12) ebenfalls mit der Symmetrieachse des rohrförmigen Teils (21) ausgerichtet ist.

4. Vorrichtung nach Anspruch 3, **dadurch gekennzeichnet, dass** das Verbindungsstück (3) ferner einen ringförmigen Vorsprung (34) umfasst, der auf dessen Außenfläche angeordnet ist und den distalen Abschnitt (32) und den proximalen Abschnitt (31) voneinander abgrenzt, wobei der Außendurchmesser des ringförmigen Vorsprungs (34) unter Gewährleistung eines funktionellen Spiels im Wesentlichen gleich dem Innendurchmesser des rohrförmigen Körpers (21) des Rings (2) und ist und größer ist als der Durchmesser der Öffnung (222) des Flansches (22), so dass das Verbindungsstück (3) im Ring (2) entlang einer Achse, die im Wesentlichen mit der Symmetrieachse (23) des rohrförmigen Körpers (21) zusammenfällt, gleiten und den Flansch (22) bis zum Aufliegen des ringförmigen Vorsprungs (34) auf der Innenwand des Flansches (22) durchqueren kann, wobei die Symmetrieachse (33) des Verbindungsstücks dann vom ringförmigen Vorsprung (34) im Wesentlichen mit der Symmetrieachse (23) des rohrförmigen Körpers (21) zusammengehalten wird, wobei der Flansch, die Innenwand des rohrförmigen Körpers (21) des Rings und der ringförmige Vorsprung eine Sperre bilden, die die Möglichkeit einschränkt, dass flüssige oder feste Elemente über die erste Öffnung (12) in den Raum zwischen der Innen- und Außenseite gelangen.

5. Vorrichtung nach Anspruch 4, **dadurch gekennzeichnet, dass** die erste Öffnung (12) der flexiblen Ummanetelung (1) einen Durchmesser aufweist, der etwas kleiner ist als der Außendurchmesser des zylindrischen distalen Abschnitts (321) des Verbindungsstücks (3), wobei der Durchmesser derart definiert ist, dass, wenn das Verbindungsstück vom Inneren der Ummantelung (1) her in diese Öffnung (12) eingeführt wird, der Rand der ersten Öffnung (12) verformt wird und eine dem Inneren der Ummantelung (1) zugewandte ringförmige Falte (14) bildet, die die Außenfläche des zylindrischen Körpers (321) des distalen Abschnitts (32) des Verbindungsstücks (3) umgibt, wobei diese Flate ein Gelenk bildet, das mit der Sperre, die vom Flansch (22) des Rings (2), der Innenwand des rohrförmigen Körpers (21) des Rings (2) und dem ringförmigen Vorsprung (34) des Verbindungsstücks (3) zusammenwirkt, um eine dichte Barriere auf Höhe der ersten Öffnung (12) der Ummantelung (1) zu bilden.

6. Vorrichtung nach einem der Ansprüche 1 - 5, **dadurch gekennzeichnet, dass** der Körper des Werkzeugs (5) auf Höhe des Endes (51) mit dem Kopplungselement (52) kegelförmig ist und das Ende des rohrförmigen Rings (2) eine kegelförmige Öffnung (211) aufweist, die derart dimensioniert ist, dass, wenn das Ende (51) des Werkzeugs in den Ring (2) eingeführt wird, diese Einführung eine feste Steckverbindung des Rings mit dem Ende (51) des Werkzeugs (5) zur Folge hat, die jede relative Verschiebung und/oder Rotation der Ummantelung (1) in Bezug auf das Werkzeug (5) blockiert.

7. Vorrichtung nach Anspruch 6, **dadurch gekennzeichnet, dass** die Wand des Endes (51) des Körpers des Werkzeugs (5) einen Längsvorsprung (512) aufweist, der eine Passfeder bildet, und die Innenwand des rohrförmigen Rings (2) einen Längsausschnitt (212) umfasst, der zur Aufnahme der Passfeder (512) dimensioniert ist, wobei die Einrichtung aus Passfeder und Ausschnitt ein zusätzliches Mittel zur Blockierung relativer Rotationen der Ummantelung (1) in Bezug auf das Werkzeug (5) bildet.

8. Vorrichtung nach einem der vorstehenden Ansprüche, **dadurch gekennzeichnet, dass** das Kopplungselement (52) des Werkzeugs (5) einen Hohlraum (512) bildet, der einen hexagonalen hinteren Bereich (523) und ein kegelstumpfförmiges vorderes Ende (522) umfasst, und der proximale Abschnitt (31) des Verbindungsstücks (3) einen Stecker mit hexagonalem Querschnitt bildet, der derart dimensioniert ist, dass er mit minimalem Spiel in den hexagonalen Hohlraum (521) des Kopplungselements (52) eingesteckt werden kann, wobei die Außenwand des proximalen Abschnitts (31) einen kegelstumpfförmigen Bereich (315) umfasst, der an der Wand angeordnet und zum Zusammenwirken mit dem kegelstumpfförmigen vorderen Ende (522) des Kopplungselements (52) ausgebildet ist, um so die Koaxialität der beiden Elemente im Einsatz zu wahren.

9. Vorrichtung nach Anspruch 8, **dadurch gekennzeichnet, dass** die Innenwand des Hohlraums (521) des Kopplungselemenets (52) des Werkzeugs (5) auf Höhe des hexagonalen hinteren Bereichs (523) einen abgekanteten Hals (524) umfasst, der Körper (311) des proximalen Abschnitts (31) des Verbindungsstücks (3) abgekantete Wölbungen (312) umfasst, die derart ausgebildet sind, dass sie im abgekanteten Hals (524) untergebracht werden und eine Schnappverriegelung des Körpers (311) des proximalen Abschnitts (31) des Verbindungsstücks (3) im Kopplungselement (52) gewährleisten können.

10. Vorrichtung nach einem der Ansprüche 8 oder 9, **dadurch gekennzeichnet, dass** der vom Kopplungselement (52) des Werkzeugs (5) ausgebildete Hohlraum eine bilnde zylindrische Bohrung (526) umfasst, die den Boden des Hohlraums bildet und deren Symmetrieachse mit der allgemeinen Symmetrieachse des Kopplungselements (52) zusammenfällt, wobei der Körper (311) des proximalen Abschnitts (31) des Verbindungsstücks (3) an seinem Ende ein in Längsrichtung gespaltenes zylindrisches Endelement (33) umfasst, dessen Durchmesser derart definiert ist, dass dessen Einführung in die zylindrische Bohrung (526) mit minimalem Spiel erfolgt.

11. Vorrichtung nach Anspruch 10, **dadurch gekennzeichnet, dass** die Außenwand des Endelements (33) des Körpers (311) des proximalen Abschnitts (31) des Verbindungsstücks (3) Wölbungen (332) umfasst, die Rippen bilden, so dass der Querschnittsdurchmesser des Endelements (33) lokal größer ist als der Durchschnitt der Bohrung (526) des Kopplungselements (52) des Werkzeugs (5) und die Aufnahme des Endelements (33) des Körpers (311) des proximalen Abschnitts (31) des Verbindungsstücks ohne Spiel erfolgt.

12. Vorrichtung nach einem der vorstehenden Ansprüche, **dadurch gekennzeichnet, dass** das distale Ende (32) des Verbindungsstücks (3) eine hexagonale Einprägung (323) aufweist, die die Aufnahme des proximalen Endes eines perkutanen chirurgischen Instruments ermöglicht, wobei dieses proximale Ende ein Passstück mit hexagonalem Schnitt umfasst.

13. Vorrichtung nach einem der vorstehenden Ansprüche, **dadurch gekennzeichnet, dass** der zylindrische Körper (321) des distalen Abschnitts (32) des Verbindungsstücks (3) einen ringförmigen Hals (322) umfasst, der zur Aufnahme eines Orientierungsrings (6) geeignet ist, wobei der Orientierungsring derart am zylindrischen Körper (21) angeordnet ist, dass, der Orientierungsring für den Arzt sichtbar ist, wenn bei der Kopplung die dichte Barriere auf Höhe der ersten Öffnung (12) der Ummantelung (1) wirksam ist.

14. Vorrichtung nach einem der vorstehenden Ansprüche, **dadurch gekennzeichnet, dass** der rohrförmige Ring (2) einteilig ausgebildet ist.

15. Vorrichtung nach einem der vorstehenden Ansprüche, **dadurch gekennzeichnet, dass** der rohrförmige Ring (2) auf der Innenfläche der Ummantelung (1) angeordnet und an einem seiner Enden (22) derart mit der Ummantelung (1) verbunden ist, dass der rohrförmige Ring (2) insgesamt im Inneren der Ummantelung (1) angeordnet ist, wenn sich das Werkzeug in der Ummantelung (1) befindet.

16. Verfahren zur sterilen Kopplung eines Biopsieinstruments und eines Antriebswerkzeugs dieses Instruments mittels einer Kopplungsvorrichtung nach einem der Ansprüche 1 - 5, umfassend die Schritte:
- in einer Vorstufe: Zusammendrehen der Ummantelung (1), indem der Ring (2) ins Innere der Ummantelung (1) eingeschoben wird, bis dessen freies Ende (213) über die zweite Öffnung (13) der Ummantelung (1) aus der Ummantelung hervortritt;
- in einem ersten Schritt: Montieren des Kopplungselements (52) des Werkzeugs (5) am proximalen Ende (31) des Kopplungselements (3) durch das Assistenzpersonal, bis die beiden Elemente ineinander verschachtelt sind;
- in einem zweiten Schritt: Greifen und Präsentieren der zusammengedrehten Ummantelung (1) durch den Arzt, wobei das Assistenzpersonal nach der Einführung des distalen Endes (51) des Werkzeugs (5) in den Ring (2) der Ummantelung (1) das Werkzeug (5) loslässt, wobei dieses mittels der zusammengedrehten Ummantelung vom Arzt derart festgehalten wird, dass das Gewicht des Werkzeugs die Ummantelung (1) teilweise wieder in ihre auseinandergefaltete Form versetzt wird;
- in einem dritten Schritt: Abschließen der Wiederherstellung der Ummantelung (1), ohne das Werkzeug (5) zu berühren, so dass diese das Werkzeug (5) umschließt, und hermetisches Verschließen der zweiten Öffnung (13) der Ummantelung (1);
- in einem vierten Schritt: Positionieren des perkutanen chirurgischen Instruments durch Einführen des Verbindungsstücks des Instruments ins Ende (323) des Kopplungselements (3).

17. Verfahren nach Anspruch 16, **dadurch gekennzeichnet, dass** die Kopplungsvorrichtung in steriler Verpackung an den Arzt geliefert wird, wobei die Ummantelung (1) bereits zusammengedreht ist und die Vorstufe unabhängig bei der Verpackung der Kopplungsvorrichtung ausgeführt wird.

## Claims

1. A device for the sterile coupling of a percutaneous surgical instrument to a rotary driving tool (5) of said instrument, said tool (5) including an end (51) provided with a coupling endpiece (52), including:
- a flexible enclosure (1) having a circular first opening (12) and a second opening (13) making it possible to insert the driving tool (5) into said enclosure (1), the first opening (12) being positioned on the wall of the enclosure (1) such that, when the driving tool is in the pouch, the coupling endpiece (52) is placed across from said first opening (12);
- a connecting endpiece (3), including a cylindrical distal segment (32) ensuring the coupling with the percutaneous surgical instrument and a proximal segment (31) ensuring the coupling with the driving tool (5), configured to pass through the enclosure (1) through the first circular opening (12) such that the end (323) of its distal segment (32) is placed outside the enclosure (1), its proximal segment (31) being placed inside the enclosure (1);
- a tubular ring (2) forming a conduit (211) in which the connecting endpiece is intended to be inserted, positioned on the inner surface of the enclosure (1) across from the first circular opening (12), said ring (2) being secured to the enclosure (1) by one of its ends (22), its other end (213) being configured to secure the enclosure to the wall of the driving tool (5) by insertion of the end (51) of the body of the driving tool (5) into the conduit (211), said conduit (211) being configured such that the connecting endpiece (3) can rotate freely around its axis inside the ring (2) while being held axially;
the enclosure (1), the ring (2) and the connecting endpiece (3) including means that cooperate in order to form a tight barrier at the first circular opening (12), said barrier being configured so as not to hinder the rotation of the connecting endpiece (3).

2. The device according to claim 1, **characterized in that** the distal segment (32) of the connecting endpiece (3) has a tubular shape and includes a cavity (323) configured to accommodate the proximal end of a percutaneous surgical instrument, the end of the proximal segment (31) of the connecting endpiece (3) being configured to be able to be housed in the coupling endpiece (52) of the driving tool (5).

3. The device according to claim 1 or 2, **characterized in that** the ring (2) includes a disc-shaped flange (22) located at the end of the tubular part (21) by which said ring is fastened on the inner wall of the enclosure (1), the flange having a central opening (222), the center of which is located on the axis of symmetry of the tubular part (21), the ring and the flange being fastened on the inner wall of the enclosure (1) such that the center of the first opening (12) is also aligned on the axis of symmetry of the tubular part (21).

4. The device according to claim 3, **characterized in that** the connecting endpiece (3) further includes an annular protuberance (34) positioned on its outer surface and delimiting the distal segment (32) and the proximal segment (31); the outer diameter of the annular protuberance (34) being substantially equal, to within any functional play, to the inner diameter of the tubular body (21) of the ring (2) and larger than the diameter of the opening (222) of the flange (22), such that the connecting endpiece (3) can slide in the ring (2) along an axis substantially combined with the axis of symmetry (23) of the tubular body (21) and pass through the flange (22) until the annular protuberance (34) abuts on the inner wall of the flange (22), the axis of symmetry (33) of the connecting endpiece then being kept substantially combined with the axis of symmetry (23) of the tubular body (21) by the annular protuberance (34), the flange, the inner wall of the tubular body (21) of the ring and the annular protuberance forming a baffle limiting the possibility of passage of fluid or solid elements between the inside and the outside of the enclosure through the first opening (12).

5. The device according to claim 4, **characterized in that** the first opening (12) of the flexible enclosure (1) has a diameter slightly smaller than the outer diameter of the cylindrical distal segment (321) of the connecting endpiece (3), said diameter being defined such that when the connecting endpiece is inserted into this opening (12) from the inside of the enclosure (1), the edge of said first opening (12) deforms and constitutes an annular fold (14) oriented toward the outside of the enclosure (1) that surrounds the outer surface of the cylindrical body (321) of the distal segment (32) of the endpiece (3), this fold constituting a seal that cooperates with the baffle formed by the flange (22) of the ring (2), the inner wall of the tubular body (21) of the ring (2) and the annular protuberance (34) of the connecting endpiece (3) in order to constitute a tight barrier at the first opening (12) of the enclosure (1).

6. The device according to any one of claims 1 to 5, **characterized in that**, the body of the driving tool (5) having a conical shape at the end (51) provided with the coupling endpiece (52), the end of the tubular ring (2) has a conical opening (211) sized such that when the end (51) of the driving tool is inserted into the ring (2), this insertion causes a tight fitting of the ring on the end (51) of the driving tool (5) that blocks any relative translational and/or rotational movement of the enclosure (1) with respect to the driving tool (5).

7. The device according to claim 6, **characterized in that** the wall of the end (51) of the body of the tool (5) including a longitudinal protuberance (512) forming a key, the inner wall of the tubular ring (2) includes a longitudinal notch (212) dimensioned to receive said key (512), the key-notch assembly forming a complementary blocking means of the relative rotational movement of the enclosure (1) with respect to the driving tool (5).

8. The device according to any one of the preceding claims, **characterized in that**, the coupling endpiece (52) of the driving tool (5) forming a cavity (521) including a hexagonal rear zone (523) and a frustoconical front end (522), the proximal segment (31) of the connecting endpiece (3) forms a hexagonal male element, dimensioned so as to be able to be inserted, with minimal play, into the hexagonal cavity (521) of the coupling endpiece (52), the outer wall of said proximal segment (31) including a frustoconical zone (315) arranged on said wall and configured to cooperate with the frustoconical front end (522) of the coupling endpiece (52), so as to ensure the maintenance of the coaxial nature of the two elements during use.

9. The device according to claim 8, **characterized in that**, the inner wall of the cavity (521) of the coupling endpiece (52) of the driving tool (5) including a beveled groove (524) at the hexagonal rear zone (523), the body (311) of the proximal segment (31) of the connecting endpiece (3) includes beveled bosses (312) configured to be able to be housed in said beveled groove (524) and to ensure blocking by snapping of the body (311) of the proximal segment (31) of the connecting endpiece (3) in the coupling endpiece (52).

10. The device according to one of claims 8 or 9, **characterized in that**, the cavity formed by the coupling endpiece (52) of the driving tool (5) including a blind cylindrical piercing (526) forming the body of the cavity and the axis of symmetry of which is combined with the general axis of symmetry of the coupling endpiece (52), the body (311) of the proximal segment (31) of the connecting endpiece (3) includes, at its end, a cylindrical end element (33) that is slotted over its length, and the diameter of which is defined such that its insertion into the blind cylindrical piercing (526) is done with minimal play.

11. The device according to claim 10, **characterized in that** the outer wall of the end element (33) of the body (311) of the proximal segment (31) of the connecting endpiece (3) includes bosses (332) forming overthicknesses such that the diameter of the section of the end element (33) is locally larger than the diameter of the piercing (526) of the coupling endpiece (52) of the driving tool (5) and the fitting of the end element (33) of the body (311) of the proximal segment (31) of the connecting endpiece is done without play.

12. The device according to one of the preceding claims, **characterized in that** the distal end (32) [of] the connecting endpiece (3) includes a hexagonal cavity (323) making it possible to receive the proximal end of a surgical percutaneous instrument, this proximal end including a hexagonal endpiece.

13. The device according to one of the preceding claims, **characterized in that** the cylindrical body (321) of the distal segment (32) of the connecting endpiece (3) includes an annular groove (322) able to receive a reference ring (6), said reference ring being arranged on the cylindrical body (21) such that when the coupling is done, if the tight barrier at the first opening (12) of the enclosure (1) is effective, the reference ring is visible by the practitioner.

14. The device according to one of the preceding claims, **characterized in that** the tubular ring (2) is formed in a monobloc manner.

15. The device according to one of the preceding claims, **characterized in that** the tubular ring (2) is positioned on the inner surface of the enclosure (1) and secured to the enclosure (1) by one of its ends (22) such that the entire tubular ring (2) is positioned inside the enclosure (1) when the driving tool is located in the enclosure (1).

16. A method for performing the sterile coupling of a biopsy instrument and a driving tool of said instrument using a coupling device according to any one of claims 1 to 15, including the following steps:
- a preliminary step for turning the enclosure (1) over on itself, this step being done by pushing back the ring (2) inside the enclosure (1) until its free end (213) emerges from the enclosure through the second opening (13) of the enclosure (1);
- a first step during which the assistant assembles the coupling endpiece (52) of the driving tool (5) on the proximal end (31) of the connecting endpiece (3) until performing the fitting of the two elements one in the other;
- a second step during which the practitioner grasps and presents the turned over enclosure (1) and during which the assistant, after having introduced the distal end (51) of the driving tool (5) into the ring (2) of the enclosure (1), releases the driving tool (5), the latter then being held by the practitioner using the enclosure turned over on itself such that, due to the weight of the driving tool, the enclosure (1) partially regains its unfolded shape;
- a third step during which the practitioner finishes returning the enclosure (1) to its shape without touching the driving tool (5), such that it closes the driving tool (5), then hermetically seals the second opening (13) of the enclosure (1);
- a fourth step during which the practitioner places the percutaneous surgical instrument by inserting the connecting endpiece of said instrument into the end (323) of the connecting endpiece (3).

17. A method according to claim 16 **characterized in that** the coupling device is delivered to the practitioner in sterile packaging with its pre-returned envelope, the preliminary step is implemented independently at the time of packaging of the coupling device.
